# EUROPEAN PATENT SPECIFICATION

(11) **EP 0 425 540 B1**
(45) Date of publication and mention of the grant of the patent: **17.08.1994**
(21) Application number: 89908065.9
(22) Date of filing: 22.06.1989
(51) Int. Cl.: C07C 19/08, C07C 17/20, C07C 17/00

(54) **CATALYZED HYDROFLUORINATION PROCESS**
KATALYTISCHES HYDROFLUORIERUNGSVERFAHREN
PROCEDE D'HYDROFLUORATION CATALYSEE

(30) Priority: 23.06.1988 US 210553
(43) Date of publication of application: 08.05.1991
(73) Proprietor: E.I. DU PONT DE NEMOURS AND COMPANY, Wilmington Delaware 19898 (US)
(72) Inventor: RAO, V. N. Mallikarjuna, Wilmington, DE 19809 (US)
(74) Representative: Woodcraft, David Charles
(86) International application number: US8902671
(87) International publication number: WO8912617

(56) References cited:
- US-A- 2 005 707
- US-A- 4 258 225
- US-A- 4 374 289

## Description

Process for the preparation of fluorinated alkanes by contacting alkenes, preferably halogenated alkenes or halogenated alkanes with hydrogen fluoride in the presence of niobium pentachloride (NbCl₅) or niobium pentabromide (NbBr₅).

### BACKGROUND OF THE INVENTION

A. E. Feiring, Journal of Fluorine Chemistry, 13, 7-18 (1979) discloses the use of tantalum pentafluoride and niobium pentafluoride as a catalyst for the addition of hydrogen fluoride to tetra- and trichloroethene and related compounds. The catalyst is also useful for fluorine chlorine exchange reactions. However, under the conditions of the batch experiments [HF/CCl₂=CCl₂ = 2.5, temp = 150°C, reaction time = six hours] catalysts such as BF₃, TaCl₅, Ta₂O₅, CoF₃, V₂O₅, ZrCl₄', NbCl₅, HgO, and WC1₆ showed no catalytic activity for the addition of HF to tetrachloroethylene.

The use of tantalum pentafluoride and niobium pentafluoride as a catalyst for the addition of hydrogen fluoride to unsaturated compounds has been disclosed and claimed in U.S. 4,258,225.

U.S. 4,374,289 discloses the addition of HF to CHCl=CCl₂ using NbCl₅ as a catalyst to produce CH₂ClCCl₂F. Conditions for the reaction are HF/CHCl=CCl₂ = 1.5 to 3.5, temp. 50°C to 150°C, reaction time = 15 minutes to 5 hours, NbCl₅ = 0.05 to 0.2 mole/mole CHCl=CCl₂ and pressure = (11.9-17 bar) 175 to 250 psig. The reaction is conducted under moderate temperature conditions in order to avoid overfluorination.

This invention provides conditions under which NbCl₅ or NbBr₅ functions as a mild hydrofluorination catalyst and is capable of fluorinating even less reactive molecules such as CCl₂=CCl₂.

### SUMMARY OF THE INVENTION

This invention is a process for the preparation of fluorinated alkanes by contacting at a temperature from about O°C to about 185°C under substantially anhydrous conditions, one molar equivalent of a starting material selected from alkenes, preferably halogenated alkenes or halogenated alkanes of the following formulas

R¹R²C=CR³R⁴ and R⁵R⁶R⁷R⁸C

wherein
R¹, R², R³, R⁴ and R⁵, R⁶, R⁷, R⁸ are each selected from the group represented by CₓZ₂ₓ₊₁, wherein Z is H, F, Br or Cl and wherein x = 0 to 10, preferably with the proviso that in at least one of R¹, R², R³ and R⁴ and in at least one of R⁵, R⁶, R⁷ and R⁸, Z is F, Br or Cl; and
at least one of the pairs R⁵ and R⁶, R⁷ and R⁸, R⁵ and R⁷, and R⁶ and R⁸ taken together is -(CH₂)ₙ-wherein "n" is an integer from 2 to 7, with the proviso that when R⁵ and R⁷ and/or R⁶ and R⁸ are trans, "n" must be 6 or 7, and when only two of the group R⁵, R⁶, R⁷, R⁸ are combined to form a cyclic structure the remaining two groups may be CₓZ₂ₓ₊₁, wherein Z is H, F, Br or Cl and X = 0 to 10,
with HF in the presence of at least one catalyst selected from niobium pentachloride and niobium pentabromide to produce a fluorinated alkane.

Preferably, this invention is a process for the preparation of fluorinated alkanes by contacting at a temperature from about O°C to about 185°C under substantially anhydrous conditions, one molar equivalent of a starting material selected from alkenes, preferably halogenated alkenes or halogenated alkanes of the following formulas

R¹R²C=CR³R⁴ and R⁵R⁶R⁷R⁸C

wherein R¹, R², R³, R⁴, R⁵, R⁶, R⁷ are H, F, Cl, Br, or CZ₃, wherein Z is selected from at least one of the group consisting of H, F, Cl, or Br and wherein R⁸ is Br or Cl, with the proviso that when R¹ is H only two of R², R³ and R⁴ can be Cl, preferably with the proviso that at least one of R¹, R², R³ and R⁴ and at least one of R⁵, R⁶, R⁷ and R⁸ is or contains F, Cl or Br, equivalent with HF, the mole ratio of HF to alkene being from 4 to 30 and the mole ratio of HF to halogenated alkane being from 1 to 30, in the presence of 0.001 to 5 molar equivalent, preferably 0.001 to 0.250 molar equivalent of at least one catalyst selected from NbCl₅ and NbBr₅.

### DETAILS OF THE INVENTION

The resulting fluorinated alkane produced in accordance with this invention has one or more fluorine atoms over and above the number of fluorine atoms originally present in the alkene or the alkane used as the starting material.

The alkene or alkane starting materials of the invention do not substantially react with hydrogen fluoride alone under the conditions of temperature and pressure used in this invention and require the presence of added catalyst specifically NbCl₅ or NbBr₅. Preferred alkene of the formula R¹R²C=CR³R⁴ is wherein R¹, R², R³, and R⁴ are Cl, i.e. tetrachloroethylene, Cl₂C=CCl₂.

The preferred halogenated alkanes of the formula R⁵R⁶R⁷R⁸C are wherein R⁷ is CHCl₂, CH₂Cl, CH₃, Cl, or H, when R⁵, R⁶, and R⁸ are Cl; and wherein R⁵ and R⁶ are H when R⁷ and R⁸ are Cl. The specifically preferred halogenated alkanes are CCl₃CHCl₂, CCl₃CH₂Cl, CCl₃CH₃, CCl₄, CHCl₃, and CH₂Cl₂.

The reaction can be carried out in the liquid phase or vapor phase and at autogenous pressures or under constant pressure ranging from atmospheric to superatmospheric. Both the liquid phase and vapor phase processes include batch, semicontinuous and continuous modes of operation.

The NbCl₅ or NbBr₅ are used in an amount from 0.001 to 5 moles, preferably 0.001 to 0.250 moles, per mole of starting alkene or halogenated alkane for reasons of economy and effectiveness. The catalyst is a commercially available crystalline solid and can be used alone or on a support such as carbon.

The reaction can be carried out at from O°C to 185°C. The preferred temperature is from 35°C to 165°C.

Anhydrous or substantially anhydrous conditions means that water, which is detrimental to the reaction, should be excluded as much as possible from the reaction zone. The HF which is commercially available as anhydrous grade can be used in the reaction directly. Exclusion of moisture from the reaction vessel by means of appropriate moisture traps or other means is a routine procedure and is well known in the art.

1 to 30 molar equivalents of HF, relative to the halogenated alkane, are utilized for reaction with halogenated alkanes and 4 to 30 molar equivalents of HF, relative to the alkene, be utilized for reaction with alkenes. At least 5 molar equivalents of HF is preferred, particularly for highly chlorinated alkenes such as CCl₂=CCl₂. In practice from 15 to 30 molar equivalents of HF ensures the best combination of economics and fluorination yield.

The reaction vessel is constructed from materials which are resistant to the action of hydrogen halide such as nickel alloys including monel, "Hastelloy" and "Inconel".

The liquid phase reactions are conducted by introducing the reagents in any order into the reaction vessel. Generally, the NbCl₅ or NbBr₅ and starting alkene or halogenated alkane are placed in the reaction vessel which is then cooled, and the required amount of hydrogen fluoride is condensed in the vessel. The vessel may be evacuated prior to the introduction of hydrogen fluoride and cooled in Dry Ice or liquid nitrogen to facilitate addition of the hydrogen fluoride. The contents of the vessel are raised to the appropriate reaction temperature and agitated by shaking or stirring for a length of time sufficient to cause the reaction to occur.

For liquid phase reactions, the amount of NbCl₅ or NbBr₅ used is from 0.001 to 5 moles, preferably 0.001 to 0.250 mole, per mole of starting alkene or halogenated alkane, and is more preferably from 0.005 to 1 mole, and in some cases 0.005 to 0.1 mole per mole of starting alkene or halogenated alkane. The amount of HF used in the reaction is from 1 to 30 molar equivalents per mole of halogenated alkane and from 1 to 30, preferably 4 to 30 molar equivalents per mole of alkene. The reaction can be carried out at from O°C to 185°C. The preferred temperature is 35°C to 165°C. The reaction time can be from 0.5 to 18 hours; the preferred times are from 1 to 8 hours.

In the vapor phase reaction, the reactants are introduced into the reactor above their boiling points. The temperature of the reactor must also be sufficient to keep the products of the reaction in the vapor state so that they pass over into a cooled receiver beyond the reactor rather than remain in the catalyst zone for a prolonged period of time.

For vapor phase reactions, it is convenient to support the NbCl₅ or NbBr₅ on an inert porous material such as carbon or other known supports. The amount of catalyst to inert support is from 10% to 50% with amounts of about 25% being preferred. The amount of HF used in the reaction is from 1 to 30 molar equivalents per mole of halogenated alkane and from 4 to 30 molar equivalents per mole of alkene. The reaction can be carried out at from 50°C to 185°C. The preferred temperature is 70°C to 170°C. The contact time of the reagents with the catalyst may be specified instead of reaction time. The combined operations of feed rate, control of reactor temperature and pressure and rate of removal of product from the reactor influence the residence time of the product in the reactor. It may be desirable to shorten the residence time for a given product within the reactor to control the formation of undesired products. Contact time is the average time that the reactant product mixture is in contact with the catalyst. Broadly, contact times of from 0.1 to 25 seconds are useful with preferred contact times in the range of 1 to 10 seconds.

Under the reaction conditions set forth above a portion of the NbCl₅ or NbBr₅ may be in the form of NbCl₅₋ₓFₓ or NbBr₅₋ₓFₓ.

Pressure is not critical. Atmospheric, superatmospheric and autogenous pressures are the most convenient and are therefore preferred.

The fluorinated alkanes produced by the invention have utility as refrigerants, solvents and blowing agents.

### EXAMPLES

### General Experimental Procedure

The reactor consisted of a 100 ml high pressure cylinder made of monel or "Inconel" containing a magnetic stirrer and an internal thermocouple. Mounted on top of the reactor was a condenser and a back pressure regulator connected to an optional on line analytical system. Suitable inlet and exit lines were also present to allow for admission of reactants and withdrawal of products.

To the reactor was charged NbCl₅ in the desired amount. The reactor was then cooled and evacuated. The alkene or halogenated alkane starting material and the required amount of HF were then admitted to the reactor. It was then pressurized with nitrogen to the desired pressure while still cold and then gradually brought to the desired operating temperature with stirring by using external heat provided by an oil bath. The back pressure regulator was set to the desired operating pressure prior to heating the reactor.

At the completion of the reaction, the product was isolated by conventional means and analyzed by gas chromatography. All of the percentages reported in the Examples are area %.

### EXAMPLE 1

The General Experimental Procedure was followed using 16.5 g of CCl₂=CCl₂, 3.0 of NbCl₅ and 15 g of anhydrous HF [Molar ratio of HF/CCl₂=CCl₂ = 7.5/1]. The reactor was pressurized to 13.6 bar (200 psig) when cold with nitrogen and the back pressure regulator was set for 34 bar (500 psig). The contents were heated with stirring at 142-148°C for about 2 hours. Product analysis indicated 85.0% CClF₂CHCl₂ and 10.9% CF₃CHCl₂, in addition to small amounts of other organics.

### EXAMPLE 2

The General Experimental Procedure was followed using 20.25 g of CHCl₂CCl₃, 3.0 g of NbCl₅ and 15 g of anhydrous HF [Molar ratio of HF/CHCl₂CCl₃ = 7.5/1]. The reactor was pressurized to 13.6 bar (200 psig) when cold with nitrogen and the back pressure regulator was set for 34 bar (500 psig). The contents were heated with stirring at 142-148°C for three hours. Product analysis indicated 92.5% CClF₂CHCl₂ and 5.1% CF₃CHCl₂, and small amounts of other organics.

### EXAMPLE 3

The General Experimental Procedure was followed using 30.8 g of CCl₄, 3.0 g of NbCl₅ and 10 g of anhydrous HF [Molar ratio of HF/CCl₄ = 2.5/1]. The reactor was pressurized to 13.6 bar (200 psig) when cold with nitrogen and the back pressure regulator was set for 27.2 bar (400 psig). The contents were heated with stirring at 80-85°C for about 45 minutes. An off gas analysis during this period showed greater than 98% CF₂Cl₂. The pressure was carefully vented to remove most of the CF₂Cl₂. The product remaining in the reactor (24.0g) was analyzed and found to contain 1.3% CF₂Cl₂, 21.2% CFCl₃ and 77.5% of CCl₄.

### EXAMPLE 4

The General Experimental Procedure was followed using 34.0 g CH₃CCl₃, 0.3 g NbCl₅ and 5.0 g anhydrous HF [Molar ratio of HF/CH₃CCl₃ = 1/1]. The reactor was pressurized to 6.8 bar (100 psig) when cold with nitrogen and the back pressure regulator was set for 13.6 bar (200 psig). The contents were heated with stirring at 36-37°C for about 30 minutes. Product analysis showed 7.6% CH₃CClF₂, 46.3% CH₃CCl₂F and 43.5% CH₃CCl₃ in addition to small amounts of other organics.

### EXAMPLE 5

Example 4 was substantially repeated except that 0.5 g NbCl₅ was used. Product analysis indicated 12.1% CH₃CClF₂, 57.3% CH₃CCl₂F, and 28.2% CH₃CCl₃ in addition to small amounts of other organics.

## Claims

1. A process for the preparation of fluorinated alkanes by contacting at a temperature from about O°C to about 185°C under substantially anhydrous conditions, one molar equivalent of a starting material selected from alkenes or halogenated alkanes of the following formulas
R¹R²C=CR³R⁴ and R⁵R⁶R⁷R⁸C
wherein R¹, R², R³, R⁴, R⁵, R⁶, R⁷ are H, F, Cl, Br, or CZ₃, wherein Z is selected from at least one of H, F, Cl, or Br, and
wherein R⁸ is Br or Cl, with the proviso that when R¹ is H, only two of R², R³ and R⁴ can be Cl. with HF, the mole ratio of HF to alkene being from 4 to 30 and the mole ratio of HF to halogenated alkane being from 1 to 30,
in the presence of 0.001 to about 5 molar equivalent of at least one catalyst selected from NbCl₅ and NbBr₅.

2. The process of Claim 1 wherein the starting material is Cl₂C=CCl₂.

3. The process of Claim 1 wherein the starting material is selected from CCl₃CHCl₂, CCl₃CH₂Cl, CCl₃CH₃, CCl₄, CHCl₃ and CH₂Cl₂.

4. The process of Claim 1 wherein the catalyst is NbCl₅.

5. The process of Claim 1 wherein the temperature is from about 35°C to about 165°C.

6. The process of Claim 1 wherein the mole ratio of HF to starting material is from 15 to 30.

7. The process of Claim 1 carried out in the presence of 0.001 to 0.250 molar equivalent of at least one catalyst selected from NbCl₅ and NbBr₅.

8. A process for the preparation of fluorinated alkanes by contacting at a temperature from about O°C to about 185°C under substantially anhydrous conditions, one molar equivalent of a starting material selected from alkenes or halogenated alkanes of the following formulas
R¹R²C=CR³R⁴ and R⁵R⁶R⁷R⁸C
wherein
R¹, R², R³, R⁴ and R⁵, R⁶, R⁷, R⁸ are each selected from the group represented by CₓZ₂ₓ₊₁, wherein Z is H, F, Br or Cl and wherein x = 0 to 10, with the proviso that in at least one of R¹, R², R³ and R⁴ and in at least one of R⁵, R⁶, R⁷ and R⁸, Z is F, Br or Cl; and
at least one of the pairs R⁵ and R⁶, R⁷ and R⁸, R⁵ and R⁷, and R⁶ and R⁸ taken together is -(CH₂)ₙ-wherein "n" is an integer from 2 to 7, with the proviso that when R⁵ and R⁷ and/or R⁶ and R⁸ are trans, "n" must be 6 or 7, and when only two of the group R⁵, R⁶, R⁷, R⁸ are combined to form a cyclic structure the remaining two groups may be CₓZ₂ₓ₊₁, wherein Z is H, F, Br or Cl and x = 0 to 10,
with HF, the mole ratio of HF to halogenated alkane being from 1 to 30 and the mole ratio of HF to alkene being from 4 to 30, in the presence of 0.001 to about 5 molar equivalent of at least one catalyst selected from niobium pentachloride and niobium pentabromide.

## Patentansprüche

1. Verfahren zur Herstellung fluorierter Alkane durch Zusammenbringen bei einer Temperatur von etwa 0 °C bis etwa 185 °C unter im wesentlichen wasserfreien Bedingungen von einem Moläquivalent eines Ausgangsmaterials, ausgewählt aus Alkenen oder halogenierten Alkanen der folgenden Formeln:
R¹R²C=CR³R⁴ und R⁵R⁶R⁷R⁸C
worin R¹, R², R³, R⁴, R⁵, R⁶, R⁷ für H, F, Cl, Br oder CZ₃ stehen, worin Z ausgewählt wird aus wenigstens einem von H, F, Cl oder Br, und worin R⁸ für Br oder Cl steht, mit der Maßgabe, daß, wenn R¹ für H steht, nur zwei von R², R³ und R⁴ Cl sein können, mit HF, wobei das Molverhältnis von HF zu Alken 4 bis 30 beträgt, und das Molverhältnis von HF zu halogeniertem Alkan 1 bis 30 beträgt, in Gegenwart von 0,001 bis etwa 5 Moläquivalenten wenigstens eines Katalysators, ausgewählt aus NbCl₅ und NbBr₅.

2. Verfahren nach Anspruch 1, bei dem das Ausgangsmaterial Cl₂C=CCl₂ ist.

3. Verfahren nach Anspruch 1, bei dem das Ausgangsmaterial ausgewählt wird aus CCl₃CHCl₂, CCl₃CH₂Cl, CCl₃CH₃, CCl₄, CHCl₃ und CH₂Cl₂.

4. Verfahren nach Anspruch 1, bei dem der Katalysator NbCl₅ ist.

5. Verfahren nach Anspruch 1, bei dem die Temperatur etwa 35 °C bis etwa 145 °C beträgt.

6. Verfahren nach Anspruch 1, bei dem das Molverhältnis von HF zu Ausgangsmaterial 15 bis 30 beträgt.

7. Verfahren nach Anspruch 1, das in Gegenwart von 0,001 bis 0,250 Moläquivalenten wenigstens eines Katalysators, ausgewählt aus NbCl₅ und NbBr₅, durchgeführt wird.

8. Verfahren zur Herstellung fluorierter Alkane durch Zusammenbringen bei einer Temperatur von etwa 0 °C bis etwa 185 °C unter im wesentlichen wasserfreien Bedingungen von einem Moläquivalent eines Ausgangsmaterials, ausgewählt aus Alkenen oder halogenierten Alkanen der folgenden Formeln:
R¹R²C=CR³R⁴ und R⁵R⁶R⁷R⁸C
worin R¹, R², R³, R⁴ und R⁵, R⁶, R⁷, R⁸ jeweils ausgewählt werden aus der Gruppe, dargestellt durch CₓZ₂ₓ₊₁, worin Z für H, F, Br oder Cl steht, und worin x = 0 bis 10, mit der Maßgabe, daß in wenigstens einem von R¹, R², R³ und R⁴ und wenigstens einem von R⁵, R⁶, R⁷ und R⁸ Z für F, Br oder Cl steht,
und daß wenigstens eines der Paare R⁵ und R⁶, R⁷ und R⁸, R⁵ und R⁷ und R⁶ und R⁸ zusammengenommen -(CH₂)ₙ- bedeutet, worin "n" eine ganze Zahl von 2 bis 7 bedeutet, mit der Maßgabe, daß, wenn R⁵ und R⁷ und/oder R⁶ und R⁸ in trans-Form vorkommen, "n" 6 oder 7 sein muß und wenn nur zwei der Gruppe R⁵, R⁶, R⁷, R⁸ unter Bildung einer cyclischen Struktur kombiniert werden, die restlichen beiden Gruppen CₓZ₂ₓ₊₁ sein können, worin Z für H, F, Br oder Cl steht und x = 0 bis 10,
mit HF, wobei das Molverhältnis von HF zu halogeniertem Alkan 1 bis 30 beträgt und das Molverhältnis von HF zu Alken 4 bis 30 beträgt, in Gegenwart von 0,001 bis etwa 5 Moläquivalenten von wenigstens einem Katalysator, ausgewählt aus Niobpentachlorid und Niobpentabromid.

## Revendications

1. Un procédé pour la préparation d'alcanes fluorés par mise en contact à une température d'environ 0°C à environ 185°C, dans des conditions sensiblement anhydres, d'un équivalent molaire d'un produit de départ choisi parmi les alcènes et les alcanes halogénés des formules suivantes
R¹R²C=CR³R⁴ et R⁵R⁶R⁷R⁸C
où R¹, R², R³, R⁴, R⁵, R⁶, R⁷ sont H, F, Cl, Br ou CZ₃, Z étant choisi parmi au moins l'un des éléments H, F, Cl et Br, et où R⁸ est Br ou Cl, étant entendu que lorsque R¹ est H, seuls deux des radicaux R², R³ et R⁴ peuvent être des atomes de chlore,
avec HF, le rapport molaire de HF à l'alcène étant de 4 à 30 et le rapport molaire de HF à l'alcane halogéné étant de 1 à 30,
en présence de 0,001 à environ 5 équivalents molaires d'au moins un catalyseur choisi parmi NbCl₅ et NbBr₅.

2. Le procédé de la revendication 1 dans lequel le produit de départ est Cl₂C=CCl₂.

3. Le procédé de la revendication 1 dans lequel le produit de départ est choisi parmi CCl₃CHCl₂, CCl₃CH₂Cl, CCl₃CH₃, CCl₄, CHCl₃ et CH₂Cl₂.

4. Le procédé de la revendication 1 dans lequel le catalyseur est NbCl₅.

5. Le procédé de la revendication 1 dans lequel la température est d'environ 35 à environ 165°C.

6. Le procédé de la revendication 1 dans lequel le rapport molaire de HF au produit de départ est de 15 à 30.

7. Le procédé de la revendication 1 mis en oeuvre en présence de 0,001 à 0,0250 équivalent molaire d'au moins un catalyseur choisi parmi NbCl₅ et NbBr₅.

8. Un procédé pour la préparation d'alcanes fluorés par mise en contact à une température d'environ 0 à environ 185^{.}°C dans des conditions sensiblement anhydres d'un équivalent molaire d'un produit de départ choisi parmi les alcènes et les alcanes halogénés des formules suivantes
R¹R²C=CR³R⁴ et R⁵R⁶R⁷R⁸C
dans lesquelles
R¹, R², R³, R⁴, et R⁵, R⁶, R⁷, R⁸ sont choisis chacun dans le groupe représenté par CₓZ₂ₓ₊₁, où Z est H, F, Br, ou Cl et où x = 0 à 10, étant entendu que dans l'un au moins des radicaux R¹, R², R³ et R⁴ et dans l'un au moins des radicaux R⁵, R⁶, R⁷ et R⁸, Z est un atome de fluor, de brome ou de chlore ; et
l'une au moins des paires de radicaux R⁵ et R⁶, R⁷ et R⁸, R⁵ et R⁷, et R⁶ et R⁸ pris en semble est -(CH₂)ₙ-, où n est un nombre entier de 2 à 7, étant entendu que lorsque R⁵ et R⁷ et/ou R⁶ et R⁸ sont de la forme trans, n doit valoir 6 ou 7, et que lorsque seuls deux des groupes R⁵, R⁶, R⁷, R⁸ sont combinés pour former une structure cyclique, les deux groupes restants peuvent être CₓZ₂ₓ₊₁, où Z est H, F, Br, ou Cl et où x = 0 à 10,
avec HF, le rapport molaire de HF à l'alcane halogéné étant de 1 à 30 et le rapport molaire de HF à l'alcène étant de 4 à 30, en présence de 0,001 à environ 5 équivalents molaires d'au moins un catalyseur choisi parmi le pentachlorure de niobium et le pentabromure de niobium.
